# EUROPÄISCHE PATENTSCHRIFT

(11) **EP 1 663 939 B1**
(45) Veröffentlichungstag und Bekanntmachung des Hinweises auf die Patenterteilung: **30.12.2009**
(21) Anmeldenummer: 04741073.3
(22) Anmeldetag: 16.07.2004
(51) Int. Cl.: C07C 68/06, C07C 68/00

(54) **VERFAHREN ZUR HERSTELLUNG VON ORGANISCHEN CARBONATEN**
METHOD FOR THE PRODUCTION OF ORGANIC CARBONATES
PROCEDE DE PRODUCTION DE CARBONATES ORGANIQUES

(30) Priorität: 11.09.2003 DE 10341951
(43) Veröffentlichungstag der Anmeldung: 07.06.2006
(73) Patentinhaber: Lurgi GmbH, 60439 Frankfurt am Main (DE)
(72) Erfinder: BUCHOLD, Henning, 63452 Hanau (DE); EBERHADT, Jürgen, 63110 Rodgau (DE); WAGNER, Ulrich, 06408 Biendorf (DE); WÖLK, Hans-Jürgen, 83022 Rosenheim (DE)
(74) Vertreter: Meyer-Dulheuer, Karl-Hermann
(86) Internationale Anmeldenummer: PCT/EP2004/007913
(87) Internationale Veröffentlichungsnummer: WO 2005/028415

(56) Entgegenhaltungen:
- EP-A- 0 041 622
- EP-A- 0 478 073
- EP-A- 0 638 541
- EP-A- 0 866 051

## Beschreibung

Gegenstand der Erfindung ist ein Verfahren zur Herstellung von, organischen Carbonaten aus einer Mischungen von Polyalkoholen und deren Carbonaten und Carbamaten.

Organische Carbonate, wie Dimethylcarbonat und Diphenylcarbonat sind als Zwischenprodukte der chemischen Industrie für eine Vielzahl von Synthesen von größter Bedeutung.

So ist Dimethylcarbonat ein Ausgangsmaterial für aromatische Polycarbonate. Dimethylcarbonat wird mit Phenol zum Diphenylcarbonat umgeestert und in einer Schmelzpolymerisation mit Bisphenol zum aromatischen Polycarbonat umgesetzt (Daniele Delledonne; Franco Rivetti; Ugo Romano: "Developments in the production and application of dimethylcarbonate" Applied Catalysis A: General 221 (2001) 241 - 251). Dimethylcarbonat kann zur Verbesserung der Oktanzahl von Benzin eingesetzt werden und umweltproblematische Zuschlagstoffe wie MTBE ersetzen (Michael A. Pacheco; Christopher L. Marshall: "Review of Dimethyl Carbonate (DMC) Manufacture and it's Characteristics as a Fuel Additive" Energy & Fuels 11 (1997) 2 - 29). Dabei ist vor allem die leichte biologische Abbaubarkeit, die Ungiftigkeit und die gute Anwendbarkeit als Zuschlagstoff in Benzin zu nennen. Dimethylcarbonat hat eine Reihe von Anwendungen in der chemischen Synthese. Bei Temperaturen bei oder unter der Siedetemperatur von 90°C kann Dimethylcarbonat als Methoxylierungsmittel verwendet werden. Bei höheren Temperaturen um 160°C lässt sich Dimethylcarbonat als Methylierungsmittel einsetzen (Pietro Tundi; Maurizio Selva: "The Chemistry of Dimethyl Carbonate" Acc. Chem. Res. 35 (2002) 706 - 716).

Die bis ca. 1980 gebräuchliche Methode zur Herstellung von Dimethylcarbonat war die Alkoholyse von Phosgen mit Methanol (US 2,379,740, Pittsburgh Plate Glass Company 1941) oder (Kirk-Othmer, Encyclopedia of Chemical Technology, 3rd Edition, Volume 4, 758). Die Toxizität des Phosgens und das Entstehen von korrosivem Chlorwasserstoff stehen einer umweltbewussten kommerziellen Verwendung im großen Maßstab allerdings entgegen.

Der zur Zeit hauptsächlich eingesetzte Prozess ist die Umsetzung von Methanol mit Kohlenmonoxid und Sauerstoff an einem Kupferchloridkontakt, beschrieben in US 5,210,269 von Enichem (1993). Diese oxidative Carbonylierung verläuft über Kupfermethoxychlorid und eine anschließende Reaktion mit Kohlenmonoxid zu Dimethylcarbonat. Das Hauptproblem dieses Prozesses ist die Deaktivierung des Katalysators durch Wasser. Der desaktivierte Katalysator muss aufwendig regeneriert oder der Wassergehalt im Reaktor klein gehalten werden.

Eine Variante der oxidativen Carbonylierung ist eine zweistufige Reaktion über Methylnitrit. In einem Vorreaktor wird Methylnitrit aus Methanol, Stickstoffmonoxid und Sauerstoff synthetisiert, wobei Wasser als Nebenprodukt entsteht. Nach Entfernung des Wassers wird gasförmiges Methylnitrit in einem Festbettreaktor an einem Palladiumchloridkatalysator mit CO zu Dimethylcarbonat umgesetzt; das entstehende NO wird im Kreis geführt. Dieses Verfahren hat den Nachteil, dass der Umgang mit korrosivem Stickstoffmonoxid gefährlich ist.

Eine weitere Möglichkeit zur Herstellung von Dimethylcarbonat ist die Umesterung eines zyklischen Carbonats mit Methanol. Verfahren mit Ethylen- oder Propylencarbonat als Ausgangsmaterial sind bekannt (US 4,734,518 Texaco 1988; US 4,691,041 Texaco 1987). Ausgehend vom zyklischen Carbonat kann durch eine Umesterung mit Methanol das Dimethylcarbonat und gleichzeitig jeweils ein Mol des entsprechenden Diols synthetisiert werden. Die Alkylencarbonate lassen sich einfach darstellen. Der Nachteil dieser Methode ist die Coproduktion von Diolen bei der Herstellung des Dimethylcarbonats.

Die direkte Alkoholyse von Harnstoff mit Methanol ist eine weitere Möglichkeit zur Herstellung von Dimethylcarbonat. Die Synthese verläuft in zwei Schritten über den Carbamatsäuremethylester zum Dimethylcarbonat. Die Reaktionsgeschwindigkeit wird durch das gebildete Ammoniak stark gehemmt. Zur verbesserten Synthese wurden daher chemische und physikalische Methoden vorgeschlagen, um das entstehende Ammoniak zu entfernen.

Auch eine Fällung des entstehenden Ammoniaks durch BF₃ wurde erfolgreich durchgeführt (US 2,834,799; 1958), ist jedoch angesichts der entstehenden Salzfrachten unwirtschaftlich.

Das Entfernen des Ammoniaks (US 4,436,668; BASF1984) durch Zugabe von inertem Gas in einer zweiten Stufe lieferte bislang nur ungenügende Umsätze und Selektivitäten. Zur Verbesserung des Prozesses wurde eine zweite Stufe mit einem Katalysator-Reaktanden Dialkyl-isozyanat-alkoxyzinn (US 5,565,603; Exxon 1996; US 5,561,094; Exxon 1996) eingesetzt, der durch Methanol in situ hergestellt wird. Als Nachteil ist die Bereitstellung und Aufarbeitung des Katalysator-Reaktanden zu nennen.

Eine Alternative zur direkten Synthese ist der Einsatz eines zyklischen Carbonats (US 5,489,702 Mitsubishi Gas Chemical 1996; US 5,349,077; Mitsubishi Gas Chemical 1994). Hier wird in einem ersten Schritt ein Diol mit Harnstoff umgesetzt und ein zyklisches Alkylencarbonat mit 5 bzw. 6 Ringatomen synthetisiert. Im zweiten Prozessschritt wird das Alkylencarbonat mit Methanol umgeestert. Das Diol kann anschließend im Kreislauf gefahren werden.

Die bei der Alkoholyse hergestellten Zwischenprodukte müssen anschließend mit Methanol umgeestert werden, um das Dimethylcarbonat als Produkt zu erhalten. Die Umesterung ist eine katalysierte Reaktion. Als heterogene Katalysatoren werden basische Alkali- und Erdalkalimetalle bzw. Oxide eingesetzt. Beispiele für Alkali- oder Erdalkalimetalle in Zeolithen sind in US 6,365,767 der Exxon, Beispiele für Metalloxide sind in US 6,207,850 Mobil Oil genannt. Verfahren zur Umesterung von Ethylen- und Propylencarbonaten mit Alkoholen in Gegenstrom-Festbett-Rohrreaktoren mit homogenen oder heterogenen Katalysatoren (US 5,231,212; Bayer 1993; US 5,359,118; Bayer 1994) sowie ein Verfahrenspatent zur Synthese über Epoxide mit anschließender Umesterung an bifunktionellen Katalysatoren (US 5,218,135; Bayer 1993) sind ebenfalls bereits bekannt. Die Umesterung von zyklischen Carbonaten mit Alkoholen in einer Reaktivdestillation ist beschrieben (US 6,346,638; Asahi Kasei Kabushiki Kaisha 2002). Eine Reaktivextraktion mit Kohlenwasserstoffen oder Benzin als Phase zur Aufnahme des Dimethylcarbonats und einer polaren Phase aus Alkylencarbonat zur Aufnahme der Alkohole ist aus der US 5,489,703 bekannt.

Nur wenige dieser prinzipiell möglichen Synthesewege sind für eine technische und wirtschaftliche Realisierung aussichtsreich. Bei geforderten großen Mengen an Dimethylcarbonat kommen nur solche Verfahren in Betracht, die auch die notwendigen Rohstoffe in genügender Menge preiswert zur Verfügung haben. In den letzten Jahren wurde deshalb verstärkt daran gearbeitet, die Herstellung von organischen Carbonaten, vorzugsweise Dimethylcarbonat, basierend auf Harnstoff und Methanol technisch durchzuführen. Trotz zahlreicher Entwicklungen besitzen die bisher beschriebenen Verfahren zum Teil erhebliche Nachteile, so dass eine elegante technische Route zur Gewinnung von organischen Carbonaten wie DMC noch aussteht.

Als nachteilig erweist sich bei den bisher beschriebenen Verfahren:
- Die Reaktion von Harnstoff mit Methanol verläuft über die Zwischenstufe des Carbamates.
- Während der Reaktion wird Ammoniak abgespalten, welches entfernt werden muss.
- Die Reaktion verläuft wegen der unzureichenden Ammoniakabtrennung nur zu geringen Umsatzgraden.
- Ammoniak kann prinzipiell durch verschiedene Methoden aus dem Reaktionsgemisch entfernt werden, jedoch wird bei den aus dem Stand der Technik bekannten Verfahren hierbei ein zu entsorgender Feststoff gebildet oder ein großer Teil des eingesetzten Methanols mit ausgetragen.
- Große Mengen von Methanol müssen im Kreislauf gefahren werden.
- Ein für DMC entwickeltes Verfahren lässt sich nicht ohne Weiteres auf die Synthese anderer Carbonate ausweiten.

Um diese Nachteile zu überwinden, wurde in der gleichzeitig eingereichten deutschen Patentanmeldung AZ 103 41 952.7-44 (internes Aktenzeichen: L 1 P 22) ein neues Zwischenprodukt und eine Synthese dieses Zwischenprodukts beschrieben, das einen so hohen Siedepunkt hat, dass es auch bei der notwendigen Austreibung des Ammoniaks durch Strippen nicht mit entfernt wird.

Dieses Zwischenprodukt ist eine Mischung verschiedener Polyalkohole und deren Carbonate und Carbamate und hat den besonderen Vorteil, dass es eine Funktion als Hilfsstoff für die Herstellung von organischen Carbonaten auch dann erfüllen kann, wenn es nicht in reiner Form, sondern als Mischung vorliegt. Durch Einsatz dieser Mischung kann sowohl die Löslichkeit für den Harnstoff wie auch das Siedeverhalten den Erfordernissen der Ammoniakentfernung entsprechend eingestellt werden. Die Verwendung dieses Zwischenproduktes zur Herstellung von organischen Carbonaten ist Gegenstand der vorliegenden Anmeldung.

Das erfindungsgemäße Verfahren zur Herstellung von organischen Carbonaten besteht darin, dass eine Mischung von Polyalkoholen und deren organischen Carbonaten und Carbamaten, die durch Umsetzung von Harnstoff, einem substituierter Harnstoff, einem Salz oder Ester der Carbamidsäure oder einem ihrer N-substituierten Derivate (Alkyl-, Aryl-Reste wie Methyl-, Ethyl-, Phenyl-, Benzyl-) mit polymeren mehrfunktionalen Alkoholen wie Polyalkylenglykolen, Polyester-Polyolen oder Polyether-Polyolen der allgemeinen Formel I 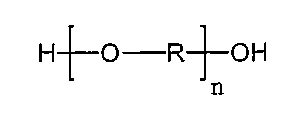 in dem R eine geradkettige oder verzweigte Alkylengruppe mit 2 bis 12 Kohlenstoffatomen und n eine Zahl zwischen 2 und 20 bedeuten,
oder vollständig oder teilweise hydrolysierten Polyvinylalkoholen der allgemeinen Formel II 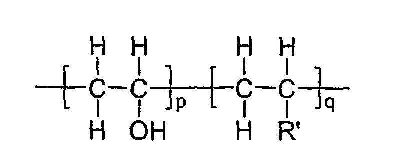 in der R' eine Alkyl-, Aryl- oder Acylgruppe mit 1 bis 12 Kohlenstoffatomen, p und q Zahlen zwischen 1 bis 20 bedeuten

oder mit Mischungen dieser Verbindungen, ohne oder in Gegenwart eines die Ammoniakabspaltung begünstigenden alkalischen Katalysators gewonnen und der dabei frei werdende Ammoniak oder das Amin aus der Reaktionsmischung durch ein Strippgas und/oder Dampf und/oder Vakuum entfernt wurde, mit einem Alkohol oder einem Phenol unter Bildung organischer Carbonate und Rückbildung der polymeren Polyalkohole der Formeln I oder II umgesetzt wird, die im Kreislauf wieder zur Herstellung einer Mischung von organischen Carbonaten oder Carbamaten durch Umsetzung mit Harnstoff, substituierten Harnstoffen, Salzen oder Estern der Carbamidsäure oder einem ihrer N-substituierten Derivate verwendet werden.

In dem erfindungsgemäßen Verfahren werden zur Herstellung von Dimethylcarbonat und/oder anderen Alkylcarbonaten als Alkohole Methylalkohol und/oder geradkettige oder verzweigte, aliphatische Alkohole mit 2 bis 10 Kohlenstoffatomen eingesetzt.

Zur Herstellung von Diphenylcarbonat und/oder Arylcarbonaten werden Phenol und/oder substituierte Phenole verwendet, die 1 bis 4 Kohlenstoffatome aufweisende Alkylgruppen tragen.

Die erfindungsgemäße Umsetzung der Mischung von polymeren Alkoholen und deren Carbonaten bzw. Carbamaten mit einem Alkohol oder einem Phenol unter Bildung organischer Carbonate wird in vorteilhafter Weise bei Temperaturen zwischen 10°C und 270°C durchgeführt. Hierbei wird in Gegenwart von Katalysatoren gearbeitet. Hierfür sind alkalisch reagierende Salze, Oxide, Hydroxide, Alkoholate der ersten und zweiten Hauptgruppe oder der 1. Bis 8. Nebengruppe des Periodensystems, basische Zeolithe oder polymere Ionenaustauscher als Katalysatoren geeignet. Beispielsweise sind Magnesium- oder Zinkkatalysatoren, die sowohl als Oxid oder auch als Acetat eingesetzt werden können katalytisch wirksam.

Bei Temperaturen zwischen 10°C und 270°C °C erfolgt eine zügige Umsetzung der Mischung der organischen Carbonate und Carbamate mit aliphatischen oder araliphatischen Alkoholen oder Phenolen. Vorteilhaft ist die Anwendung eines leicht erhöhten Drucks von ca. 6 bar und einer Temperatur von etwa 140°C. Das Gleichgewicht ist nach weniger als einer Stunde im Batchbetrieb eingestellt.

Die nach der Aufarbeitung zurückgebildeten polymeren Polyalkohole der Formeln I and II werden im Kreislauf zurückgeführt und erneut zur Herstellung einer Mischung von organischen Carbonaten oder Carbamaten eingesetzt.

## Patentansprüche

1. Verfahren zur Herstellung eines organischen Carbonats aus einer Mischung von polymeren Polyalkoholen und deren Carbonaten und Carbamaten, die durch Umsetzung von Harnstoff, einem substituierten Harnstoff, einem Salz oder Ester der Carbamidsäure oder einem ihrer N-substituierten Derivate mit einem Polyalkylenglykol, Polyester-Polyol oder einem Polyether-Polyol der allgemeinen Formel I 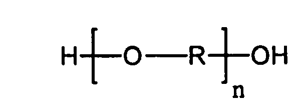 in dem R eine geradkettige oder verzweigte Alkylengruppe mit 2 bis 12 Kohlenstoffatomen und n eine Zahl zwischen 2 und 20 bedeuten, oder
mit einem vollständig oder teilweise hydrolysierten Polyvinylalkohol der allgemeinen Formel II 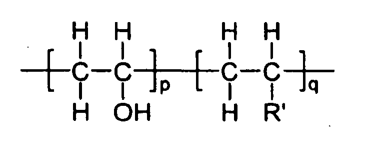 in der R' eine Alkyl-, Aryl- oder Acylgruppe mit 1 bis 12 Kohlenstoffatomen, p und q Zahlen zwischen 1 bis 20 bedeuten, oder mit Mischungen dieser Verbindungen, ohne oder in Gegenwart eines die Ammoniakabspaltung begünstigenden, alkalischen Katalysators und Abtrennung des dabei frei werdenden Ammoniaks oder des Amins aus der Reaktionsmischung durch ein Strippgas und/oder Dampf und/oder Vakuum erhältlich ist, **dadurch gekennzeichnet, dass** diese Mischung mit Alkoholen oder Phenolen unter Bildung von deren Carbonaten und Rückbildung der polymeren Polyalkohole der Formeln I der II umgesetzt wird.

2. Verfahren nach Anspruch 1, **dadurch gekennzeichnet, dass** alkalisch reagierende Salze, Oxide, Hydroxide, Alkoholate mit Elementen der Gruppen Ia, Ib, IIa, IIb, IIIa, IIIb, IVa, IVb, Va, Vb, VIb, VIIb, VIIb, des Periodensystems, basische Zeolithe, polymere Ionenaustauscher oder Tetraalkylammoniumsalze oder Triphenylphosphine oder tertiäre Amine als Katalysatoren eingesetzt werden.

3. Verfahren nach den Ansprüchen 1 bis 2, **dadurch gekennzeichnet, dass** die zurückgebildeten polymeren Polyalkohole der Formeln I oder II im Kreislauf wieder zur Herstellung einer Mischung von organischen Carbonaten oder Carbamaten eingesetzt werden.

4. Verfahren zur Herstellung von Dimethylcarbonat und/oder anderer organischer Carbonate nach den Ansprüchen 1 bis 3, **dadurch gekennzeichnet, dass** als Alkohol Methylalkohol und/oder geradkettige oder verzweigte, aliphatische Alkohole mit 2 - 10 Kohlenstoffatomen eingesetzt werden.

5. Verfahren zur Herstellung von Diphenylcarbonat und/oder Arylcarbonaten nach den Ansprüchen 1 bis 3, **dadurch gekennzeichnet, dass** Phenol und/oder substituierte Phenole, die 1 bis 4 Kohlenstoffatome aufweisende Alkylgruppen tragen, und/oder aromatische Alkohole, die 6 bis 20 Kohlenstoffatome aufweisen, und/oder Heteroatome enthaltende Alkohole und/oder eine Mischung dieser Stoffe verwendet werden.

## Claims

1. A method for producing an organic carbonate from a mixture of polymeric polyalcohols and carbonates and carbamates thereof, said mixture being obtainable by reacting urea, a substituted urea, a salt or ester of carbamic acid or one of its N-substituted derivatives with a polyalkylene glycol, polyester polyol or a polyether polyol of general formula I 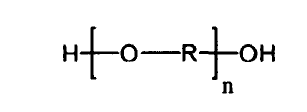 wherein R represents a straight-chain or branched alkylene group having 2 to 12 carbon atoms and n represents a number between 2 and 20; or
with a fully or partially hydrolyzed polyvinyl alcohol of general formula II 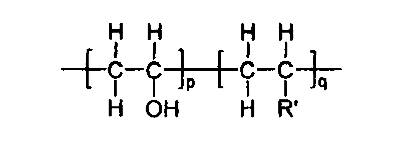 wherein R' represents an alkyl, aryl or acyl group having 1 to 12 carbon atoms, p and q represent numbers between 1 and 20; or with mixtures of these compounds, without or in the presence of an alkaline catalyst promoting ammonia elimination, and separating the ammonia released in the process or the amine from the reaction mixture by a stripping gas and/or steam and or vacuum, **characterized in that** said mixture is reacted with alcohols or phenols to form carbonates thereof and re-form the polymeric polyalcohols of formulas I or II.

2. The method according to claims 1, **characterized in that** alkaline-reacting salts, oxides, hydroxides, alkoxides with elements of groups Ia, Ib, IIa, IIb, IIIa, IIIb, IVa, IVb, Va, Vb, VIb, VIIB, VIIIb of the periodic table, basic zeolites, polymeric ion exchangers or tetraalkylammonium salts or triphenylphosphines or tertiary amines are used as catalysts.

3. The method according to claims 1 to 2, **characterized in that** the re-formed polymeric polyalcohols of formulas I or II are recycled to be used again for producing a mixture of organic carbonates or carbamates.

4. The method for producing dimethyl carbonate and/or other organic carbonates according to claims 1 to 3, **characterized in that** methyl alcohol and/or straight-chain or branched, aliphatic alcohols having 2 - 10 carbon atoms are used as alcohol.

5. The method for producing diphenyl carbonate and/or aryl carbonates according to claims 1 to 3, **characterized in that** phenol and/or phenols carrying alkyl groups having 1 to 4 carbon atoms and/or aromatic alcohols having 6 to 20 carbon atoms and/or alcohols containing heteroatoms and/or a mixture of these substances are used.

## Revendications

1. Procédé de préparation d'un carbonate organique à partir d'un mélange de polyalcools polymères et de leurs carbonates et carbamates pouvant être obtenu par la réaction de l'urée, d'une urée substituée, d'un sel ou d'un ester de l'acide carbamique ou de l'un de leurs dérivés N-substitués avec un polyalkylèneglycol, polyol de polyester ou un polyol de polyéther répondant à la formule générale I 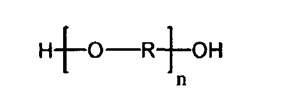 dans laquelle R représente un groupe alkylène linaire ou ramifiés renfermant 2 à 12 atomes de carbone et n représente un nombre entre 2 et 20, ou
avec un alcool polyvinylique partiellement ou complètement hydrolysé qui répond à la formule générale II 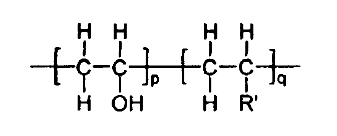 dans laquelle R' représente un groupe alkyle, aryle ou acyle renfermant 1 à 12 atomes de carbone et p et q des nombres compris entre 1 et 20, ou avec des mélanges desdits composés, en absence ou en présence d'un catalyseur alcalin favorisant la libération d'ammoniaque, ledit ammoniaque libéré ou l'amine étant éliminé(e) du mélange réactionnel par un gaz d'entraînement et/ou par de la vapeur et/ou par application d'un vide, **caractérisé en ce que** l'on fait réagir ledit mélange avec des alcools ou des phénols pour ainsi former leurs carbonates et récupérer les polyalcools polymères des formules I et II.

2. Procédé selon la revendication 1, **caractérisé en ce que** des sels, oxydes, hydroxydes, alcoolates à réaction alcaline, formés avec les éléments issus des groupes Ia, Ib, IIa, IIb, IIIa, IIIb, IVa, IVb, Va, Vb, VIb, VIIB, VIIIb du tableau périodique, des zéolithes alcalins, des échangeurs d'ions polymères ou des sels de tétraalkylammonium ou des triphénylphosphines ou des amines tertiaires sont mis en oeuvre en tant que catalyseurs.

3. Procédé selon les revendications 1 à 2, **caractérisé en ce que** les polyalcools polymères récupérés répondant aux formules I ou II sont recyclés afin de préparer de nouveau un mélange de carbonates ou carbamates organiques.

4. Procédé de préparation de carbonate de diméthyle et/ou d'autres carbonates organiques selon les revendications 1 à 3, **caractérisé en ce que** l'on met en oeuvre, en tant qu'alcool, de l'alcool méthylique et/ou des alcools aliphatique linaires ou ramifiés renfermant 2 à 10 atomes de carbone.

5. Procédé de préparation de carbonate de diphényle et/ou de carbonate d'aryle selon les revendications 1 à 3, **caractérisé en ce que** l'on utilise du phénol et/ou des phénols substitués pourvus de groupes alkyle renfermant 1 à 4 atomes de carbone et/ou des alcools aromatiques présentant 6 à 20 atomes de carbone et/ou des alcools contentant des hétéroatomes et/ou un mélange desdits composés.
